# EUROPEAN PATENT SPECIFICATION

(11) **EP 1 000 025 B1**
(45) Date of publication and mention of the grant of the patent: **21.02.2007**
(21) Application number: 97929797.5
(22) Date of filing: 04.06.1997
(51) Int. Cl.: C07D 209/02, C07D 209/44, C07D 209/82, C07D 231/00, C07D 233/02, C07D 401/00, C07D 405/00, C07D 413/00, G02B 5/23, G02B 27/00, C07D 311/92

(54) **SUBSTITUTED NAPHTHOPYRANS**
SUBSTITUIERTE NAPHTHOPYRANE
NAPHTOPYRANS SUBSTITUES

(43) Date of publication of application: 17.05.2000
(73) Proprietor: TRANSITIONS OPTICAL, INC., Pinellas Park, FL 33782 (US)
(72) Inventor: KUMAR, Anil, Pittsburgh, PA 15239 (US); KNOWLES, David, B., Apollo, PA 15613 (US); VAN GEMERT, Barry, Murrysville, PA 15668 (US)
(74) Representative: polypatent
(86) International application number: PCT/US1997/009687
(87) International publication number: WO 1998/055457

(56) References cited:
- WO-A-95/16215
- US-A- 3 567 605
- US-A- 3 627 690
- US-A- 5 066 818
- US-A- 5 238 931
- US-A- 5 238 981
- US-A- 5 274 132
- US-A- 5 451 344
- US-A- 5 466 398
- US-A- 5 514 817

## Description

### DESCRIPTION OF THE INVENTION

The present invention relates to certain novel naphthopyran compounds. More particularly, this invention relates to novel photochromic naphthopyran compounds and to compositions and articles containing such novel naphthopyran compounds. When exposed to light radiation involving ultraviolet rays, such as the ultraviolet radiation in sunlight or the light of a mercury lamp, many photochromic compounds exhibit a reversible change in color. When the ultraviolet radiation is discontinued, such a photochromic compound will return to its original color or colorless state.

Various classes of photochromic compounds have been synthesized and suggested for use in applications in which a sunlight-induced reversible color change or darkening is desired. U.S. Patent 3,567,605 (Becker) describes a series of pyran derivatives, including certain benzopyrans and naphthopyrans. These compounds are described as derivatives of chromene and are reported to undergo a color change, e.g., from colorless to yellow-orange, on irradiation by ultraviolet light at temperatures below about -30°C. Irradiation of the compounds with visible light or upon raising the temperature to above about 0°C is reported to reverse the coloration to a colorless state.

U.S. Patent 5,066,818 describes various 3,3-diaryl-3H-naphtho[2,1-b]pyrans as having desirable photochromic properties, i.e., high colorability and acceptable fade, for ophthalmic and other applications. Also disclosed by way of comparative example in the '818 patent are the isomeric 2,2-diaryl-2H-naphtho[1,2-b]pyrans, which are reported to require unacceptably long periods of time to fade after activation.

U.S. Patent 3,627,690 describes photochromic 2,2-di-substituted-2H-naphtho[1,2-b]pyran compositions containing minor amounts of either a base or weak-to-moderate strength acid. The addition of either an acid or base to the naphthopyran composition is reported to increase the fade rate of the colored naphthopyrans, thereby making them useful in eye protection applications such as sunglasses. It is reported therein further that the fade rate of 2H-naphtho-[1,2-b]pyrans without the aforementioned additives ranges from several hours to many days to reach complete reversion. U.S. Patent 4,818,096 discloses a blue coloring photochromic benzo- or naphthopyran having at the position alpha to the oxygen of the pyran ring a phenyl group having a nitrogen containing substituent in the ortho or para positions.

WO 95/16215 discloses photochromic 2H-naphtho[1,2-b]pyrans with a 5-carboxyl or amido substituent and having an acceptable fade rate, high activated intensity and a high coloration rate that are useful in photochromic articles such as ophthalmic lens.

The present invention relates to novel substituted 2H-naphtho[1,2-b]pyran compounds which have been unexpectedly found to have an acceptable fade rate in addition to a high activated intensity and a high coloration rate. In particular, the use of certain substituents at the 5-position of the naphtho-portion of the naphthopyran compound increases the fade rate without the addition of acids or bases. In addition, these compounds have certain substituents at the 2-position of the pyran ring. Certain substituents may also be present at the number 6, 7, 8, 9 or 10 carbon atoms of the naphtho portion of the naphthopyran.

### DETAILED DESCRIPTION OF THE INVENTION

In recent years, photochromic plastic materials, particularly plastic materials for optical applications, have been the subject of considerable attention. In particular, photochromic ophthalmic plastic lenses have been investigated because of the weight advantage they offer, vis-a-vis, glass lenses. Moreover, photochromic transparencies for vehicles, such as cars and airplanes, have been of interest because of the potential safety features that such transparencies offer.

In accordance with the present invention, it has now been discovered that certain novel 2H-naphtho[1,2-b]pyran compounds having an acceptable fade rate, high activated intensity and a high coloration rate may be prepared. These compounds may be described as naphthopyrans having certain substituents at the 2 position of the pyran ring and at the number 5 carbon atom of the naphtho- portion of the naphthopyran ring. Certain substituents may also be present at the 6, 7, 8, 9 or 10 carbon atoms of the naphtho portion of the naphthopyran ring. These compounds may be represented by the following graphic formula:

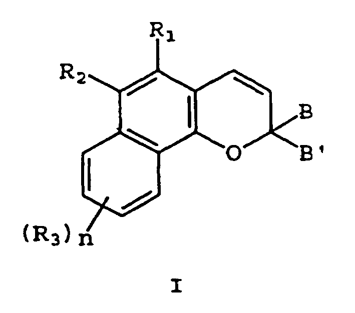

In graphic formula I, R₁ is the group, -C(O)W, W being -OR₄ or -N(R₅)R₆, wherein R₄ is hydrogen, allyl, C₁-C₆ alkyl, e.g., methyl, ethyl, propyl, butyl, pentyl, and hexyl, phenyl, mono(C₁-C₆)alkyl substituted phenyl, mono(C₁-C₆)-alkoxy-substituted phenyl, phenyl(C₁-C₃)alkyl, mono(C₁-C₆)alkyl substituted phenyl(C₁-C₃)alkyl, mono(C₁-C₆)alkoxy substituted phenyl(C₁-C₃)alkyl, C₁-C₆alkoxy(C₂-C₄)alkyl, or C₁-C₆ haloalkyl; and R₅ and R₆ may each be selected from the group consisting of hydrogen, C₁-C₆ alkyl, C₅-C₇ cycloalkyl, phenyl and mono- or di-substituted phenyl. The phenyl substituents may be C₁-C₆ alkyl and C₁-C₆ alkoxy and the halo substituent may be chloro or fluoro.

Preferably, R₁ is the group, -C(O)W, W being the groups -OR₄ or -N(R₅)R₆, wherein R₄ is hydrogen, C₁-C₄ alkyl, phenyl, mono(C₁-C₄)alkyl substituted phenyl, mono(C₁-C₄)alkoxy substituted phenyl, phenyl(C₁-C₂)alkyl, mono(C₁-C₄)alkyl substituted phenyl(C₁-C₂)alkyl, mono(C₁-C₄)alkoxy substituted phenyl(C₁-C₂)alkyl, mono(C₁-C₄)alkoxy(C₂-C₃)alkyl or C₁-C₄ haloalkyl; and R₅ and R₆ may each be selected from the group consisting of hydrogen, C₁-C₄ alkyl, C₅-C₇ cycloalkyl, phenyl and mono- or di-substituted phenyl. The phenyl substituents may be selected from C₁-C₄ alkyl and C₁-C₄ alkoxy, and the halo substituent may be chloro or fluoro.

More preferably, R₁ is the group -C(O)W, W being the group -OR₄ or -N(R₅)R₆, wherein R₄ is hydrogen, C₁-C₃ alkyl, phenyl, mono(C₁-C₃)alkyl substituted phenyl, mono(C₁-C₃)alkoxy substituted phenyl, mono(C₁-C₃)alkoxy(C₂-C₃)alkyl or C₁-C₃ haloalkyl, and wherein R₅ and R₆ are each selected from the group consisting of hydrogen, C₁-C₃ alkyl, C₅-C₇ cycloalkyl, phenyl and mono-substituted phenyl. The phenyl substituents may be selected from the group consisting of C₁-C₃ alkyl and C₁-C₃ alkoxy, and the halo substituent is fluoro. Most preferably, R₁ is the group, -C(O)W, W being -OR₄ or -N(R₅)R₆, wherein R₄ is hydrogen or C₁-C₃ alkyl, R₅ and R₆ are each hydrogen, C₁-C₃ alkyl or phenyl.

R₂ and each R₃ in graphic formula I may be hydrogen, C₁-C₆ alkyl, C₃-C₇ cycloalkyl, substituted or unsubstituted phenyl, the group -OR₇, wherein R₇ is hydrogen, C₁-C₆ alkyl, phenyl(C₁-C₃)alkyl, mono(C₁-C₆)alkyl substituted phenyl(C₁-C₃)alkyl, mono(C₁-C₆)alkoxy substituted phenyl(C₁-C₃)alkyl, C₁-C₆ alkoxy(C₂-C₄)alkyl, C₃-C₇ cycloalkyl, mono(C₁-C₄)alkyl substituted C₃-C₇ cycloalkyl, C₁-C₆ haloalkyl, allyl, and the group, -CH(R₈)X, wherein X is -CN, -CF₃, halogen or -C(O)W and R₈ is hydrogen or C₁-C₆ alkyl; or R₇ is the group, -C(O)Y, wherein Y is hydrogen, C₁-C₆ alkyl, C₁-C₆ alkoxy, the substituted or unsubstituted aryl groups phenyl or naphthyl, phenoxy, C₁-C₆ mono- or di-alkyl substituted phenoxy, C₁-C₆ alkylamino, phenylamino, C₁-C₆ mono- or di-alkyl substituted phenylamino, or C₁-C₆ mono- or di-alkoxy substituted phenylamino, each of said phenyl and naphthyl substituents being selected from C₁-C₆ alkyl or C₁-C₆ alkoxy, said halogen or halo substituents being chloro or fluoro, and n is selected from the integers 0, 1, 2 or 3.

Preferably, R₂ and each R₃ are hydrogen, C₁-C₃ alkyl, C₃-C₅ cycloalkyl, substituted or unsubstituted phenyl or -OR₇, wherein R₇ is hydrogen, C₁-C₃ alkyl, or the group, -CH(R₈)X, wherein X is -CN or -C(O)W and R₈ is hydrogen or methyl; or R₇ is the group -C(O)Y wherein Y is C₁-C₃ alkyl or C₁-C₃ alkoxy, said phenyl substituents being C₁-C₃ alkyl or C₁-C₃ alkoxy and n is selected from the integers 0 and 1. More preferably, R₂ and R₃ are each selected from the group consisting of hydrogen, C₁-C₃ alkyl, phenyl and -OR₇, wherein R₇ is hydrogen or C₁-C₃ alkyl, or R₇ is the group, -C(O)Y, wherein Y is C₁-C₃ alkyl or C₁-C₃ alkoxy, and n is selected from the integers 0 and 1. Most preferably, R₂ and R₃ are each hydrogen or C₁-C₃ alkyl. In the definitions of R₁, R₂ and R₃ in graphic formula I, like letters have the same meaning unless stated otherwise.

B in graphic formula I may be the unsubstituted, mono-, di- or tri-substituted aryl groups phenyl and naphthyl, said aryl substituents may be selected from the group consisting of C₁-C₆ alkyl, C₁-C₆ alkoxy, halogen, amino, C₁-C₆ monoalkylamino, C₁-C₆ dialkylamino, i.e., di-(C₁-C₆)alkylamino, morpholino, piperidino, indolinyl, 1-imidazolidyl, 2-imidazolin-1-yl, 2-pysazolidyl, pyrazolinyl, 1-piperazinyl and pyrrolidyl, said halogen being fluoro or chloro. Preferably, B is an unsubstituted, mono- or di-substituted phenyl, the phenyl substituents being selected from the group consisting of C₁-C₄ alkyl, C₁-C₄ alkoxy, halogen, amino, C₁-C₄ monoalkylamino, C₁-C₄ dialkylamino, morpholino, piperidino, indolinyl and pyrrolidyl, said halogen being fluoro or chloro. More preferably, B is an unsubstituted, mono- or di-substituted phenyl, the phenyl substituents being selected from the group consisting of C₁-C₃ alkyl, C₁-C₃ alkoxy, fluoro, amino, C₁-C₃ monoalkylamino, C₁-C₃ dialkylamino, morpholino, and piperidino. Most preferably, B is an unsubstituted, mono- or di-substituted phenyl, the phenyl substituents being selected from the group consisting of C₁-C₂ alkyl, C₁-C₂ alkoxy, fluoro, C₁-C₂ monoalkylamino, C₁-C₂ dialkylamino, morpholino and piperidino.

B' in graphic formula I may be selected from the group consisting of
the mono-substituted aryl groups phenyl and naphthyl, said aryl substituents may be selected from the group consisting of amino, C₁-C₆ monoalkylamino, C₁-C₆ dialkylamino, morpholino, piperidino, indolinyl, 1-imidazolidyl, 2-imidazolin-1-yl, 2-pyrazolidyl, pyrazolinyl, 1-piperazinyl and pyrrolidyl.

Preferably, B' is selected from the group consisting of mono-substituted phenyl, the phenyl substituents being selected from the group consisting of amino, C₁-C₄ monoalkylamino, C₁-C₄ dialkylamino, morpholino, piperidino, indolinyl and pyrrolidyl.

More preferably, B' is selected from the group consisting of mono-substituted phenyl, the phenyl substituents being selected from the group consisting of amino, C₁-C₃ monoalkylamino, C₁-C₃ dialkylamino, morpholino and piperidino Most preferably, B' is mono-substituted phenyl, said phenyl substituents being C₁-C₂ monoalkylamino, C₁-C₂ dialkylamino, morpholino or piperidino.

Compounds represented by graphic formula I may be prepared by the following described methods. In Reaction A 1, the benzoyl derivative of a carbazole, dibenzothiophene or dibenzofuran is prepared by Friedel-Crafts methods. See the publication Friedel-Crafts and Related Reactions, George A. Olah, Interscience Publishers, 1964, Vol. 3, Chapter XXXI (Aromatic Ketone Synthesis), and "Regioselective Friedel-Crafts Acylation of 1,2,3,4-Tetrahydroquinoline and Related Nitrogen Heterocycles: Effect on NH Protective Groups and Ring Size" by Ishihara, Yugi et al, J. Chem. Soc., Perkin Trans. 1, pages 3401 to 3406, 1992; Heterocyclic Compounds, Robert C. Elderfield, 1951, Vol. 2, Chapter 3 (Dibenzofuran) and Chapter 5 (Dibenzothiophene) ; The Chemistry of Heterocyclic Compounds, H. D. Hartough and S. L. Meisel, 1954, Vol. 7, Chapter IV (Dibenzothiophene and its Derivatives); Advances in Heterocyclic Chemistry, A. R. Katritzky and A. J. Boulton, 1974, Vol. 16, Chapter V (Recent Advances in the Chemistry of Dibenzothiophenes) ; and Heterocyclic Compounds, Robert C. Elderfield, 1952, Vol. 3, Chapter 3 (The Chemistry of Carbazole).

In Reaction A 1, the compounds represented by graphic formulae III and IV are dissolved in a solvent, such as carbon disulfide or methylene chloride, in the presence of a Lewis acid, such as aluminum chloride, to form the corresponding substituted benzoyl derivative represented by graphic formula V. R represents potential phenyl substituents.

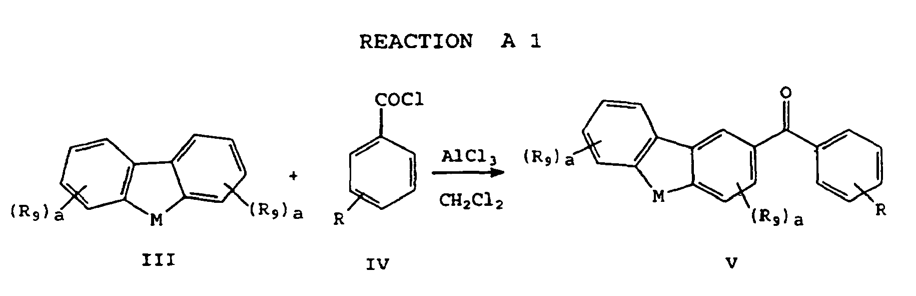

Compounds represented by graphic formula VB are either purchased or prepared, as shown in Reaction A 2, by direct amination of a commercially available fluoro substituted benzophenone represented by graphic formula VA with a primary or secondary amine compound, for example, ethyl amine, morpholine, piperidine, etc. See the publication "Nucleophilic Displacements of Activated Fluorine in Aromatic Compounds" by Bader, Henry et al, J. Org. Chem., Vol. 31, pages 2319-2321, 1966.

In Reaction A 2, the compounds represented by graphic formula VA and the amino compound are dissolved in a solvent, such as dimethyl sulfoxide (DMS), and refluxed to form the corresponding amino substituted benzophenone represented by graphic formula VB. R represents potential phenyl substituents.

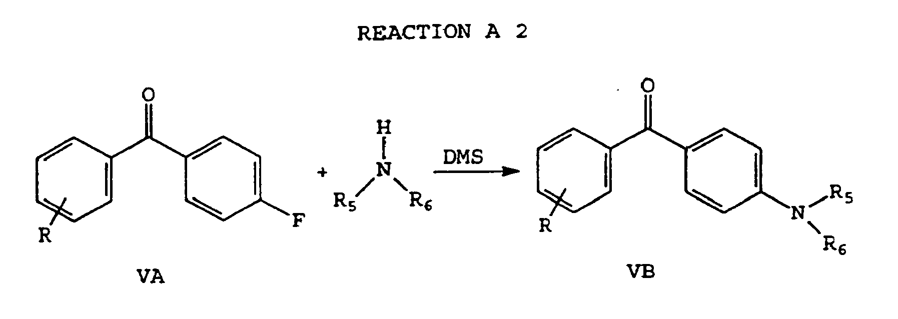

In Reaction B, the ketone represented by graphic formulae V, VB, or in more general terms VC, is reacted with sodium acetylide in a suitable solvent, such as anhydrous tetrahydrofuran (THF), to form the corresponding propargyl alcohol represented by graphic formula VI. Propargyl alcohols having B' groups represented by graphic formula II A may be prepared by the methods described in U.S. Patent 5,274,132, column 2, lines 40 to 68.

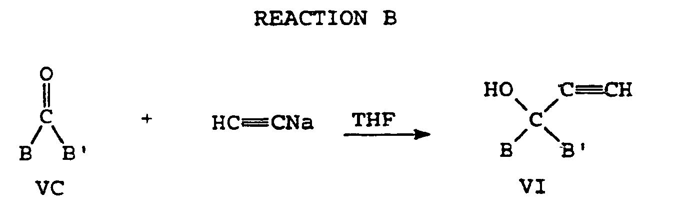

Naphthols represented by graphic formula XIIIA may be prepared by Reactions C or D. In Reaction C, substituted or unsubstituted 1,4-dihydroxy-2-naphthoic acid, represented by graphic formula VII, is reacted with methyl iodide (or ethyl iodide, propyl iodide, benzyl bromide, etc.) in the presence of ethyldiisopropyl amine in a suitable solvent such as anhydrous dimethylformamide (DMF), to form the corresponding methyl-1,4-dihydroxy-2-naphthoate, which is represented by graphic formula VIII (or XIIIA in Reaction E). This reaction is further described in J. of Org. Chem., Vol. 46(17), page 3477, 1981.

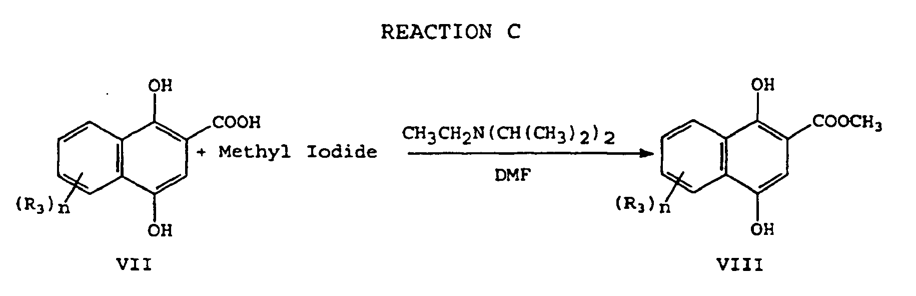

In Reaction D, a substituted or unsubstituted acetophenone, benzophenone, or benzaldehyde represented by graphic formula IX is reacted with dimethyl succinate (graphic formula X) in the presence of a base such as sodium hydride or a potassium t-butoxide in a suitable solvent such as toluene to form the appropriate substituted monoester of an a-arylidene succinic acid, represented by graphic formula XI. Other ester substituents on the compound represented by graphic formula XI may be prepared by using different succinate esters, such as diethyl succinate. Compound XI is heated with acetic anhydride and anhydrous sodium acetate to form the corresponding acetate derivative represented by the graphic formula XII. Compound XII is reacted with hydrochloric acid and an anhydrous alcohol such as anhydrous methanol to form the corresponding naphthol, represented by graphic formula XIII (or XIIIA in Reaction E). Reaction D is further described in the text Organic Reactions, Vol. VI, Chapter 1, pages 1-73, John Wiley & Sons, Inc., New York.

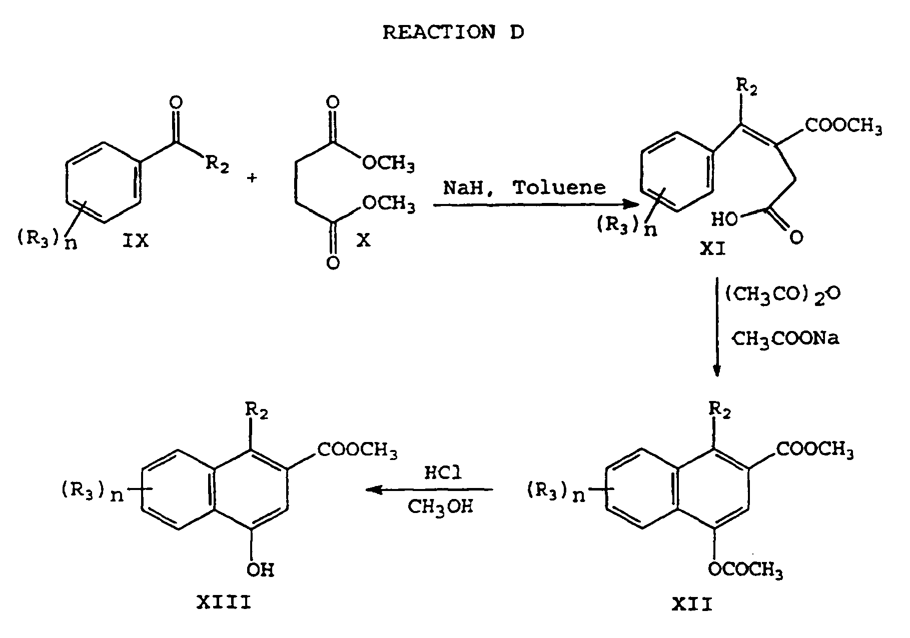

In Reaction E, the propargyl alcohol represented by graphic formula VI is coupled with the naphthol represented by graphic formula XIII A to form compounds represented by graphic formula I.

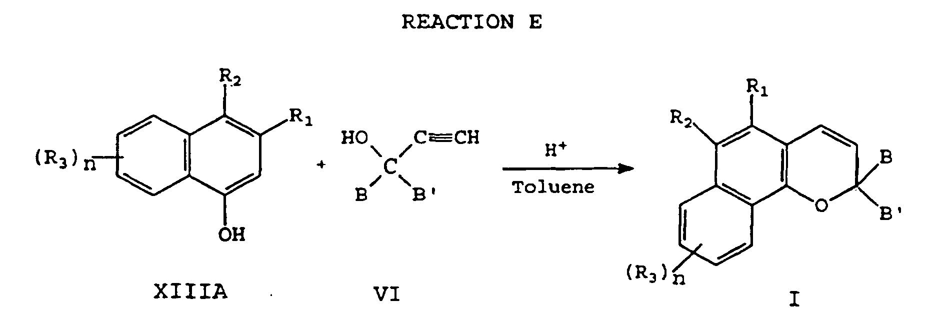

As shown in Reaction F, compounds represented by graphic formula XIV (compounds of graphic formula I in which R₂ is a hydroxy) can be converted to a variety of different groups by reaction with acylating or alkylating agents. For example, the compound represented by graphic formula XIV may be reacted with methyl iodide (or other alkylating agent) in the presence of anhydrous potassium carbonate in a suitable solvent such as anhydrous acetone to form compounds represented by graphic formula XV, in which R₂ is a methoxy substitutent. Alkylating reactions are further described in "Organic Synthesis", Vol. 31, pages 90-93, John Wiley & Sons, Inc., New York, NY. Alternatively, Compound XIV may be reacted with acetyl chloride (or other acylating agent) in the presence of triethylamine (NEt₃) in an appropriate solvent, such as methylene chloride, to form compounds represented by the graphic formula XVI, in which R₂ is an acetoxy (AcO) substitutent. Acylating reactions are further described in "Organic Synthesis," Vol. 32, pages 72-77, John Wiley & Sons, Inc., New York, NY.

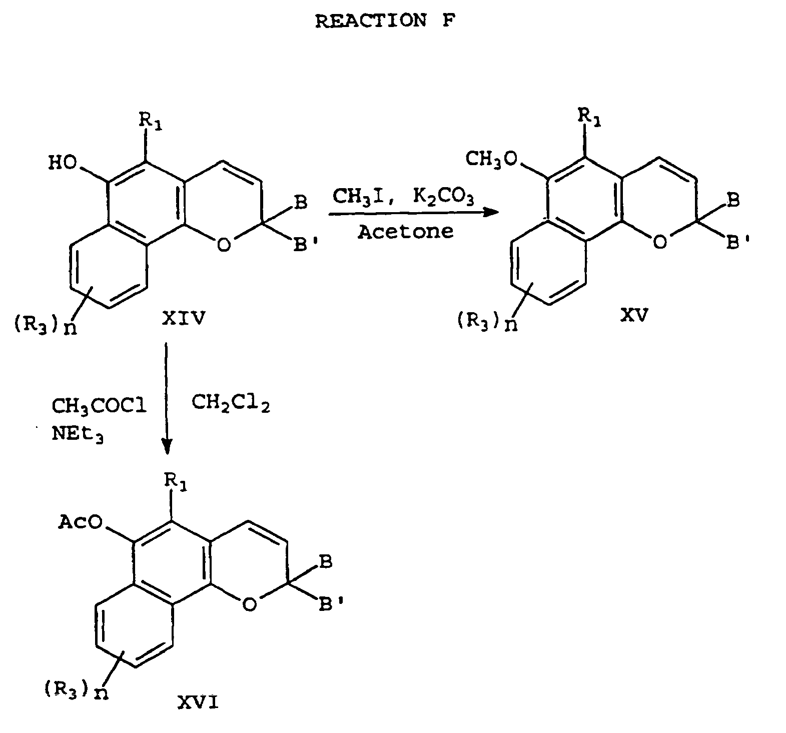

Compounds represented by graphic formula I may be used in those applications in which organic photochromic substances may be employed, such as optical lenses, e.g., vision correcting ophthalmic lenses and plano lenses, face shields, goggles, visors, camera lenses, windows, automotive windshields, aircraft and automotive transparencies, e.g., T-roofs, sidelights and backlights, plastic films and sheets, textiles and coatings, e.g., coating compositions such as paints, and verification marks on security documents, e.g., documents such as banknotes, passports and drivers' licenses for which authentication or verification of authenticity may be desired. Naphthopyrans represented by graphic formula I exhibit color changes from colorless to colors ranging from orange to blue.

Examples of contemplated naphthopyrans within the scope of the invention are the following:
(a) 2-(4-morpholinophenyl)-2-phenyl-5-methoxycarbonyl-6-hydroxy-2H-naphtho[1,2-b]pyran;
(b) 2-(4-morpholinophenyl)-2-phenyl-5-methoxycarbonyl-6-methoxy-2H-naphtho[1,2-b]pyran;
(c) 2-(4-morpholinophenyl)-2-phenyl-5-methoxycarbonyl-6-acetoxy-2H-naphtho[1,2-b]pyran;
(d) 2-(4-dimethylaminophenyl)-2-phenyl-5-methoxycarbonyl-6-acetoxy-2H-naphtho[1,2-b]pyran;
(e) 2-(4-dimethylaminophenyl)-2-phenyl-5-methoxycarbonyl-6-methyl-2H-naphtho[1,2-b]pyran; and
(f) 2,2-bis(4-dimethylaminophenyl)-5-methoxycarbonyl-6-acetoxy-2H-naphtho[1,2-b]pyran.

It is contemplated that the organic photochromic naphthopyrans of graphic formula I be used in combination with other appropriate complementary organic photochromic materials so that together they produce a near neutral gray or brown color shade when the plastic lens containing such photochromic materials are exposed to ultraviolet light. For example, a compound which colors to yellow may be blended with a compound that colors to an appropriate purple to produce a brown shade. Similarly, a compound which is orange in its colored state will produce a shade of gray when used in conjunction with an appropriate blue coloring compound. The aforesaid described combination of photochromic materials may be used also in applications other than ophthalmic lenses.

The novel naphthopyran compounds of the present invention, such as those heretofore described, may be used alone or in combination with complementary photochromic compounds, i.e., organic photochromic compounds having at least one activated absorption maxima within the range of between 400 and 700 nanometers, or substances containing same, and may be incorporated, e.g., dissolved or dispersed, in a polymeric organic host material used to prepare photochromic articles and which color when activated to an appropriate hue.

A first group of complementary organic photochromic substances contemplated for use with the organic photochromic naphthopyrans of the present invention are those having an activated absorption maximum within the visible range of greater than 570 nanometers, e.g., between about greater than 570 to 700 nanometers. These materials typically exhibit a blue, blueish-green, or blueish-purple color when exposed to ultraviolet light in an appropriate solvent or matrix. Many of such compounds are described in the open literature. For example, spiro(indoline)naphthoxazines have been described, among others, in U.S. Patent Nos. 3,562,172; 3,578,602; 4,215,010; and 4,342,668; spiro(indoline)naphthoxazines having certain substituents on the 8' and 9' positions of the naphthoxazine portion of the molecule are described in U.S. Patent 5,405,958; spiro(indoline)pyridobenzoxazines are described in U.S. Patent 4,637,698; spiro(benzindoline)pyridobenzoxazines and spiro(benzindoline)naphthoxazines are described in U.S. Patent 4,931,219; spiro(benzindoline)-naphthopyrans are described in Japanese Patent Publication 62/195383; spiro(indoline)benzoxazines are described in U.S. Patent 4,816,584; spiro(indoline)benzopyrans, spiro(indoline)naphthopyrans, and spiro(indoline)quinopyrans are described, for example, in U.S. 4,880,667; and benzopyrans and naphthopyrans having a nitrogen-containing substituent in the 2-position of the pyran ring are described in U.S. Patent 4,818,096. Spiro(indoline)pyrans are also described in the text, Techniques in Chemistry, Volume III, "Photochromism," Chapter 3, Glenn H. Brown, Editor, John Wiley and Sons, Inc., New York, 1971.

A second group of complementary organic photochromic substances contemplated for use with the organic photochromic naphthopyrans of the present invention are those having at least one absorption maximum within the visible range of between about 400 and less than 500 nanometers. These materials typically exhibit a yellow-orange color when exposed to ultraviolet light in an appropriate solvent or matrix. Such compounds include certain chromenes, i.e., benzopyrans and naphthopyrans. Many of such chromenes are described in the open literature, e.g., U.S. Patents 3,567,605; 4,826,977; and 5,066,818. Other examples of complementary benzopyrans and naphthopyrans that may be used with the naphthopyrans of the present invention include: those having a spiro adamantane group at the position alpha to the oxygen atom of the pyran ring, which are described in U.S. Patent 4,826,977; 2H-naphtho-[1,2-b]pyran compounds having certain substitutents at the number 5 and 6 carbon atoms of the naphtho portion of the naphthopyran and at the 2 position of the pyran, which are the subject of co-pending U.S. Patent Application Serial No. 08/164,187, filed December 9, 1993; 3H-naphtho[2,1-b]pyrans having at least one ortho-substituted phenyl substituent at the 3-position of the pyran ring which are described in U.S. Patent 5,066,818; 3H-naphtho[2,1-b]pyran compounds having certain substituents at the number 8 carbon atom and certain substituents at the number 7 or 9 carbon atom, all substituents being on the naphtho portion of the naphthopyran, which are the subject of co-pending U.S. Patent Application Serial No. 08/080,246, filed June 21, 1993; 3H-naphtho[2,1-b]pyrans substituted at the 3 position of the pyran ring with (i) an aryl substituent and (ii) a phenyl substituent having a 5- or 6-member heterocyclic ring fused at the number 3 and 4 carbon atoms of the phenyl substituent are described in U.S. Patent 5,384,077; diaryl-3H-naphtho[2,1-b]pyran compounds having a substituted or unsubstituted 5 or 6 member heterocyclic ring fused to the g, i, or 1 side of the naphthopyran, which are the subject of co-pending U.S. Patent Application Serial No.08/225,022, filed April 8, 1994; naphthopyran compounds substituted at the number 8 carbon atom on the naphtho portion of the naphthopyran ring, with for example, a methoxy group which are the subject of U.S. Patent 5,238,931; naphthopyran compounds, examples of which are 3-aryl-3-arylalkenyl naphthopyrans, which are described in U.S. Patent 5,274,132; and naphtho[2,1-b]pyrans substituted at the number five carbon atom with, for example, an acetoxy group, which are the subject of U.S. Patent 5,244,602.

A third group of complementary organic photochromic substances contemplated for use with the organic photochromic naphthopyrans of the present invention are those having an absorption maximum within the visible range of between about 400 to about 500 nanometers and another absorption maximum within the visible range of between 500 to 700 nanometers. These materials typically exhibit collor(s) ranging from yellow to purple and yellow/brown to purple/gray when exposed to ultraviolet light in an appropriate solvent or matrix. Examples of these compounds include certain substituted 2H-phenanthro[4,3-b]pyrans; substituted 3H-phenanthro[1,2-b]pyrans; and benzopyran compounds, such as those having substituents at the 2-position of the pyran ring and a substituted or unsubstituted heterocyclic ring, such as a benzothieno or benzofurano ring fused to the benz portion of the benzopyran. Such later described compounds are the subject of co-pending U.S. Patent Application Nos. 08/286,039 filed August 4, 1994 and U.S. Patent 5,411,679.

Photochromic articles of the present invention may contain one photochromic compound or a mixture of photochromic compounds, as desired or required. Individual photochromic compounds or mixtures of photochromic compounds may be used to attain certain activated colors such as neutral grays or browns.

The compounds of the present invention (hereinafter also referred to and included as a second group photochromic compound) may be used also in combination with the organic photochromic substances of the first complementary group of photochromic compounds described herein, i.e., those that color to blue, blueish-green, or blueish-purple or with other organic photochromic substances in the aforesaid second group of photochromic compounds. Either members of the first or second group of photochromic compounds or mixtures of such compounds may be combined with or used in conjunction with the third group of photochromic compounds described herein that exhibit colors ranging from yellow to purple and yellow/brown to purple/gray.

Each of the photochromic substances described herein may be used in amounts (or in a ratio) such that an organic host material to which the photochromic compounds or mixture of compounds is applied or in which they are incorporated exhibits a desired resultant color, e.g., a substantially neutral color when activated with unfiltered sunlight, i.e., as near a neutral color as possible given the colors of the activated photochromic compounds.

A neutral gray color exhibits a spectrum that has relatively equal absorption in the visible range between 400 and 700 nanometers. A neutral brown color exhibits a spectrum in which the absorption in the 400-550 nanometer range is moderately larger than in the 550-700 nanometer range. An alternative way of describing color is in terms of its chromaticity coordinates, which describe the qualities of a color in addition to its luminance factor, i.e., its chromaticity. In the CIE system, the chromaticity coordinates are obtained by taking the ratios of the tristimulus values to their sum, e.g., x=X/(X+Y+Z) and y=Y/(X+Y+Z). Color as described in the CIE system can be plotted on a chromaticity diagram, usually a plot of the chromaticity coordinates x and y. See pages 47-52 of Principles of Color Technology, by F. W. Billmeyer, Jr., and Max Saltzman, Second Edition, John Wiley and Sons, N.Y. (1981). As used herein, a near neutral color is one in which the chromaticity coordinate values of "x" and "y" for the color are within the following ranges (D65 illuminant): x = 0.260 to 0.400, y = 0.280 to 0.400 following activation to 40 percent luminous transmission by exposure to solar radiation (Air Mass 1 or 2).

The amount of photochromic substance or composition containing same applied to or incorporated into a host material is not critical provided that a sufficient amount is used to produce a photochromic effect discernible to the naked eye upon activation..Generally such amount can be described as a photochromic amount. The particular amount used depends often upon the intensity of color desired upon irradiation thereof and upon the method used to incorporate or apply the photochromic substances. Typically, the more photochromic substance applied or incorporated, the greater is the color intensity up to a certain limit.

The relative amounts of the aforesaid photochromic compounds used will vary and depend in part upon the relative intensities of the color of the activated species of such compounds, and the ultimate color desired. Generally, the amount of total photochromic substance incorporated into or applied to a photochromic optical host material may range from about 0.05 to about 1.0, e.g., from 0.1 to about 0.45, milligrams per square centimeter of surface to which the photochromic substance(s) is incorporated or applied. When mixtures of the aforedescribed organic photochromic complementary groups are used, the weight ratio of such materials, i.e., (first to second), (second to third), and (naphthopyran of the present invention to other second group compounds) will vary from about 1:3 to about 3:1, e.g., between about 0.75:1 and about 2:1. The combination of the first, second, and third described organic photochromic complementary groups may have a weight ratio that will vary from about 1:3:1 to 3:1:3.

The photochromic substances of the present invention may be applied to or incorporated into a host material such as a polymeric organic host material by various methods described in the art. Such methods include dissolving or dispersing the photochromic substance within the host material, e.g., casting it in place by adding the photochromic substance to the monomeric host material prior to polymerization; imbibition of the photochromic substance into the host material by immersion of the host material in a hot solution of the photochromic substance or by thermal transfer; providing the photochromic substance as a separate layer between adjacent layers of the host material, e.g., as a part of a polymeric film; and applying the photochromic substance as part of a coating placed on the surface of the host material. The term "imbibition" or "imbibe" is intended to mean and include permeation of the photochromic substance alone into the host material, solvent assisted transfer of the photochromic substance into a porous polymer, vapor phase transfer, and other such transfer mechanisms.

Compatible (chemically and color-wise) tints, i.e., dyes, may be applied to the host material to achieve a more aesthetic result, for medical reasons, or for reasons of fashion. The particular dye selected will vary and depend on the aforesaid need and result to be achieved. In one embodiment, the dye may be selected to complement the color resulting from the activated photochromic substances, e.g., to achieve a more neutral color or absorb a particular wavelength of incident light. In another embodiment, the dye may be selected to provide a desired hue to the host matrix when the photochromic substances is in an unactivated state.

Adjuvant materials may also be incorporated into the host material with the photochromic substances prior to, simultaneously with or subsequent to application or incorporation of the photochromic substances in the host material. For example, ultraviolet light absorbers may be admixed with photochromic substances before their application to the host material or such absorbers may be superposed, e.g., superimposed, as a layer between the photochromic substance and the incident light. Further, stabilizers may be admixed with the photochromic substances prior to their application to the host material to improve the light fatigue resistance of the photochromic substances. Stabilizers, such as hindered amine light stabilizers and singlet oxygen quenchers, e.g., a nickel ion complex with an organic ligand, are contemplated. They may be used alone or in combination. Such stabilizers are described in U.S. Patent 4,720,356. Finally, appropriate protective coating(s) may be applied to the surface of the host material. These may be abrasion resistant coatings and/or coatings that serve as oxygen barriers. Such coatings are known in the art.

The host material will usually be transparent, but may be translucent or even opaque. The host material need only be transparent to that portion of the electromagnetic spectrum, which activates the photochromic substance, i.e., that wavelength of ultraviolet (UV) light that produces the open form of the substance and that portion of the visible spectrum that includes the absorption maximum wavelength of the substance in its UV activated form, i.e., the open form. Preferably, the host color should not be such that it masks the color of the activated form of the photochromic substance, i.e., so the change in color is readily apparent to the observer. More preferably, the host material article is a solid transparent or optically clear material, e.g., materials suitable for optical applications, such as plano and ophthalmic lenses, windows, automotive transparencies, e.g., windshields, aircraft transparencies, plastic sheeting, polymeric films, etc.

Examples of polymeric organic host materials which may be used with the photochromic substances or compositions described herein include: polymers, i.e., homopolymers and copolymers, of polyol(allyl carbonate) monomers, diethylene glycol dimethacrylate monomers, diisopropenyl benzene monomers, and alkoxylated polyhydric alcohol acrylate monomers such as ethoxylated trimethylol propane triacrylate monomers; polymers, i.e., homopolymers and copolymers, of polyfunctional, i.e., mono-, di-, tri-, tetra, or multifunctional, acrylate and/or methacrylate monomers, polyacrylates, polymethacrylates, poly(C₁-C₁₂ alkyl methacrylates) such as poly(methyl methacrylate), polyoxy(alkylene methacrylates) such as poly(ethylene glycol bis methacrylates), poly(alkoxylated phenol methacrylates) such as poly(ethoxylated bisphenol A dimethacrylate), cellulose acetate, cellulose triacetate, cellulose acetate propionate, cellulose acetate butyrate, poly(vinyl acetate), poly(vinyl alcohol), poly(vinyl chloride), poly(vinylidene chloride), polyurethanes, thermoplastic polycarbonates, polyesters, poly(ethylene terephthalate), polystyrene, poly (alpha methylstyrene), copoly(styrene-methyl methacrylate), copoly(styrene-acrylonitrile), polyvinylbutyral and polymers, i.e., homopolymers and copolymers, of diallylidene pentaerythritol, particularly copolymers with polyol (allyl carbonate) monomers, e.g., diethylene glycol bis(allyl carbonate), and acrylate monomers.

Transparent copolymers and blends of transparent polymers are also suitable as host materials. Preferably, the host material is an optically clear polymerized organic material prepared from a thermoplastic polycarbonate resin, such as the carbonate-linked resin derived from bisphenol A and phosgene, which is sold under the trademark, LEXAN; a polyester, such as the material sold under the trademark, MYLAR; a poly(methyl methacrylate), such as the material sold under the trademark, PLEXIGLAS; polymerizates of a polyol(allyl carbonate) monomer, especially diethylene glycol bis(allyl carbonate), which monomer is sold under the trademark CR-39, and polymerizates of copolymers of a polyol (allyl carbonate), e.g., diethylene glycol bis(allyl carbonate), with other copolymerizable monomeric materials, such as copolymers with vinyl acetate, e.g., copolymers of from 80-90 percent diethylene glycol bis(allyl carbonate) and 10-20 percent vinyl acetate, particularly 80-85 percent of the bis(allyl carbonate) and 15-20 percent vinyl acetate, and copolymers with a polyurethane having terminal diacrylate functionality, as described in U.S. Patents 4,360,653 and 4,994,208; and copolymers with aliphatic urethanes, the terminal portion of which contain allyl or acrylyl functional groups as described in U.S. Patent 5,200,483; poly(vinyl acetate), polyvinylbutyral, polyurethane, polymers of members of the group consisting of diethylene glycol dimethacrylate monomers, diisopropenyl benzene monomers, and ethoxylated trimethylol propane triacrylate monomers; cellulose acetate, cellulose propionate, cellulose butyrate, cellulose acetate butyrate, polystyrene and copolymers of styrene with methyl methacrylate, vinyl acetate and acrylonitrile. More particularly, contemplated is use of the photochromic naphthopyrans of the present invention with optical organic resin monomers used to produce optically clear polymerizates, i.e., materials suitable for optical applications, such as for example plano and ophthalmic lenses, windows, and automotive transparencies. Such optically clear polymerizates may have a refractive index that may range from about 1.48 to about 1.75, e.g., from about 1.495 to about 1.66.

The present invention is more particularly described in the following examples which are intended as illustrative only, since numerous modifications and variations therein will be apparent to those skilled in the art.

### EXAMPLE 1

### STEP 1

4-Fluorobenzophenone (25 grams (gm), 0.12 moles) and morpholine (15 gm) were added to a reaction flask containing 100 milliliters of anhydrous dimethylsulfoxide, stirred and refluxed under an argon atmosphere for 8 hours. Afterwards, the contents of the reaction flask mixture was added to 500 ml of an ice-water mixture and stirred for 2 hours. The resulting solid product was filtered, washed in sequence with water followed by hexane and then air dried. The yield of the isolated product was 30 gm. A nuclear magnetic resonance (NMR) spectrum showed the product to have a structure consistent with 4-morpholinobenzophenone, which was not purified further but used directly in the next step.

### STEP 2

4-Morpholinobenzophenone (10 gm, 0.037 moles) was dissolved in a reaction flask containing 50 milliliters (ml) of anhydrous dimethylformamide saturated with acetylene and stirred at room temperature. An 18 weight percent suspension of sodium acetylide in xylene/mineral oil (0.3 mole of sodium acetylide) was added to the reaction flask and the mixture was stirred. After stirring 3 hours at room temperature under a nitrogen atmosphere, the contents of the reaction flask mixture were added to 250 ml of an ice-water mixture and stirred for one hour. The resulting solid product was filtered, washed with water followed by hexane and air dried. The yield of the isolated product was 8 gm. A nuclear magnetic resonance (NMR) spectrum showed the product to have a structure consistent with 1-(4-morpholinophenyl)-1-phenyl-2-propyn-1-ol, which was not purified further but used directly in the next step.

### STEP 3

1-(4-Morpholinophenyl)-1-phenyl-2-propyn-1-ol (about 0.025 mole) from Step 1 and methyl-1,4-dihydroxy-2-naphthoate (5 gm, 0.022 mole) were added to a reaction flask containing 200 ml of toluene and stirred. A catalytic amount of p-toluene sulfonic acid (about 100 milligrams) was added, and the mixture was stirred for 4 hours. Afterwards, the reaction mixture was poured into a 10 weight percent sodium hydroxide solution. The organic layer was separated, washed with water, and dried over anhydrous sodium sulfate. The remaining solvent, toluene, was removed under vacuum. The resulting oil was purified using a silica gel column and a 1:3 mixture of chloroform:hexane as the eluant. The photochromic fractions were combined and the eluent was removed under vacuum. The resulting product was induced to crystallize from hexane. The recovered product had a melting point of 178-179° C. A nuclear magnetic resonance (NMR) spectrum showed the product to have a structure consistent with 2-(4-morpholinophenyl)-2-phenyl-5-methoxycarbonyl-6-hydroxy-2H-naphtho[1,2-b]pyran.

### EXAMPLE 2

2-(4-Morpholinophenyl)-2-phenyl-5-methoxycarbonyl-6-hydroxy-2H-naphtho[1,2-b]pyran (2 grams) prepared as described in Example 1, anhydrous potassium carbonate (2 grams), and methyliodide (2 grams) were added to a reaction flask containing 40 milliliters of anhydrous acetone, stirred and refluxed under an argon atmosphere. Afterwards, the acetone was removed under vacuum and 25 milliliters each of water and methylene chloride were added to the reaction mixture. The mixture was stirred for 30 minutes and the organic layer was separated, washed and dried. The remaining solvent, methylene chloride, was removed under vacuum. The resulting oily concentrate was crystallized from a 1:1 hexane:diethyl ether mixture. The solid obtained was suction filtered, washed with hexane and air dried. The resulting product had a melting point of 175-176° C. A nuclear magnetic resonance (NMR) spectrum showed the product to have a structure consistent with 2-(4-morpholinophenyl)-2-phenyl-5-methoxycarbonyl-6-methoxy-2H-naphtho[1,2-b]pyran.

### EXAMPLE 3

2-(4-Morpholinophenyl)-2-phenyl-5-methoxycarbonyl-6-hydroxy-2H-naphtho[1,2-b]pyran (2 grams), prepared as described in Example 1, and triethylamine (2 grams) were added to a reaction flask containing 50 milliliters of anhydrous methylene chloride and stirred. Acetyl chloride (2 grams) was added to the reaction flask and the reaction mixture was stirred for 1 hour. Distilled water (50 milliliters) was added to the reaction flask and the reaction mixture was stirred for another half hour. Afterwards, the organic layer was separated, washed and dried over anhydrous sodium sulfate. Evaporation of solvent resulted in an oily residue that was crystallized from a 1:1 hexane:diethyl ether mixture. The solid was suction filtered, washed with hexane, and air dried. The resulting product had a melting point of 143-145° C. A nuclear magnetic resonance (NMR) spectrum showed the product to have a structure consistent with 2-(4-morpholinophenyl)-2-phenyl-5-methoxycarbonyl-6-acetoxy-2H-naphtho[1,2-b]pyran.

### EXAMPLE 4

### STEP 1

The procedure of Steeps 2 and 3 of Example 1 were followed except that in Step 2, 4-dimethylaminobenzophenone was used in place of 4-morpholinobenzophenone to produce 1-(4-dimethylaminophenyl)-1-phenyl-2-propyn-1-ol, which was used in Step 3 to produce 2-(4-dimethylaminophenyl)-2-phenyl-5-methoxycarbonyl-6-hydroxy-2H-naphtho[1,2-b]pyran.

### STEP 2

The procedure of Example 3 was followed except that 2-(4-dimethylaminophenyl)-2-phenyl-5-methoxycarbonyl-6-hydroxy-2H-naphtho[1,2-b]pyran was used in place of 2-(4-morpholinophenyl)-2-phenyl-5-methoxycarbonyl-6-hydroxy-2H-naphtho[1,2-b]pyran. The resulting product had a melting point of 156°C. A nuclear magnetic resonance (NMR) spectrum showed the product to have a structure consistent with 2-(4-dimethylaminophenyl)-2-phenyl-5-methoxycarbonyl-6-acetoxy-2H-naphtho[1,2-b]pyran.

### EXAMPLE 5

### STEP 1

The procedure of Step 3 of Example 1 was followed except that 1-(4-dimethylaminophenyl)-1-phenyl-2-propyn-1-ol was used in place of 1-(4-morpholinophenyl)-1-phenyl-2-propyn-1-ol and methyl-4-hydroxy-1-methyl-2-naphthoate was used in place of methyl-1,4-dihydroxy-2-naphthoate. The resulting product had a melting point of 182-184°C. A nuclear magnetic resonance (NMR) spectrum showed the product to have a structure consistent with 2-(4-dimethylaminophenyl)-2-phenyl-5-methoxycarbonyl-6-methyl-2H-naphtho[1,2-b]pyran.

### EXAMPLE 6

The procedure of Example 4 was followed except that in Step 1, 4,4'-bis(dimethylamino)benzophenone was used in place of 4-dimethylaminobenzophenone. The resulting product had a melting point of 222-224°C. A nuclear magnetic resonance (NMR) spectrum showed the product to have a structure consistent with 2,2-bis(4-dimethylaminophenyl)-5-methoxycarbonyl-6-acetoxy-2H-naphtho[1,2-b]pyran.

### EXAMPLE 9

### PART A

Testing was done with the photochromic naphthopyrans of the Examples incorporated or imbibed into test square polymerizates by one of two different methods. In Method I, the photochromic naphthopyrans of the Examples were incorporated into polymeric samples. The quantity of naphthopyran calculated to yield a 1.5 times 10⁻³ molal solution was added to a flask containing 50 grams of a monomer blend of 4 parts ethoxylated bisphenol A dimethacrylate (BPA 2EO DMA), 1 part poly(ethyleneglycol) 600 dimethacrylate, and 0.033 weight percent 2,2'-azobis(2-methyl propionitrile) (AIBN). The naphthopyrans were dissolved into the monomer blend by stirring and gentle heating, if necessary. After a clear solution was obtained, it was poured into a flat sheet mold having the interior dimensions of 2.2 mm x 6 inches (15.24 cm) x 6 inches (15.24 cm). The mold was sealed and placed in a horizontal air flow, programmable oven set to increase the temperature from 40°C. to 95°C. over a 5 hour interval, hold the temperature at 95°C. for 3 hours and then lower it to 60°C. for at least 2 hours before the end of the curing cycle. After the molds were opened, the polymer sheets were cut using a diamond blade saw into 2 inch (5.1 centimeters) test squares.

In Method II, the photochromic naphthopyran was imbibed into test square polymerizates prepared from a diethylene glycol bis(allyl carbonate) composition. The test squares measured 1/8 inch (0.3 centimeters) x 2 inches (5.1 centimeters) x 2 inches (5.1 centimeters). In the imbibition process, the photochromic naphthopyran was dissolved to form a 10 weight percent solution in a 1:9 mixture of ethyl cellulose:toluene. The solution was then spin coated onto the test squares and allowed to dry. Samples were then heated in a hot-air oven at 135-155°C for a period of time sufficient to thermally transfer the photochromic into the test squares. The residence time in the oven for the test squares was adjusted to imbibe comparable amounts of the naphthopyran compounds. This was done in order to yield a comparable UV absorbance at the lambda max of the compound in the near UV. After cooling, the ethyl cellulose/toluene resin film was removed from the test squares by washing with acetone.

### Part B

The photochromic test squares were tested for photochromic response rates on an optical bench. Prior to testing on the optical bench, the photochromic test squares were exposed to 365 nanometer ultraviolet light for about 15 minutes to activate the photochromic compounds and then placed into a 76°C oven for about 15 minutes to bleach or inactivate the photochromic compounds. The test squares were then cooled to room temperature, exposed to fluorescent room lighting for at least 2 hours and then kept covered for at least 2 hours prior to testing on an optical bench maintained at 75°F (23.9°C). The bench was fitted with a 150 watt Xenon arc lamp, a remote controlled shutter, a copper sulfate bath acting as a heat sink for the arc lamp, a Schott WG-320 nm cut-off filter which removes short wavelength radiation; neutral density filter(s) and a sample holder in which the square to be tested was inserted. A collimated beam of light from a tungsten lamp was passed through the square at a small angle normal to the square. After passing through the square, the light from the tungsten lamp was directed through a photopic filter attached to a detector. The photopic filter passes wavelengths such that the detector mimics the response of the human eye. The output signals from the detector(s) were processed by a radiometer.

Change in optical density (ΔOD) was determined by inserting a test square in the bleached state into the sample holder, adjusting the transmittance scale to 100%, opening the shutter from the Xenon lamp to provide ultraviolet radiation to change the test square from the bleached state to an activated (i.e., darkened) state, measuring the transmittance in the activated state, and calculating the change in optical density according to the formula Δ OD=log(100/%Ta) where %Ta is the percent transmittance in the activated state and the logarithm is to the base 10.

The Δ OD/Min, which represents the sensitivity of the photochromic compound's response to UV light, was measured over the first five (5) seconds of UV exposure, then expressed on a per minute basis. The saturation optical density (OD) was taken under identical conditions as the Δ OD/Min, except UV exposure was continued for 20 minutes for the examples in Table 1. The lambda max reported in Table 1 is the wavelength in the visible spectrum at which the maximum absorption of the activated (colored) form of the photochromic compound in a diethylene glycol bis(allyl carbonate) composition occurs. The Bleach Rate (T 1/2) is the time interval in seconds for the absorbance of the activated form of the naphthopyran in the test squares to reach one half the highest absorbance at room temperature (75°F, 23.9°C) after removal of the source of activating light. Results for the Compounds of the Examples are tabulated in Table 1.

**TABLE 1**

| | | | | |
|---|---|---|---|---|
| EXAMPLE COMPOUNDS | LAMBDA MAX (VISIBLE) | ΔOD/MIN SENSITIVITY | ΔOD@ SATURATION | Bleach (T1/2) |
| 1 | 511 | 0.64 | 1.36 | 193 |
| 2 | 541 | 0.29 | 0.95 | 292 |
| 3 | 545 | 0.24 | 0.31 | 88 |
| 4 | 591 | 0.27 | 0.30 | 70 |
| 5 | 584 | 0.20 | 0.51 | 200 |
| 6 | 618 | 0.16 | 0.07 | 19 |

All test quares were prepared by method 1.

The results of Table 1 show that a range of values for bleach rate, ΔOD at saturation, and sensitivity are obtained for the Example Compounds 1 through 8 of the present invention depending on the nature of substituents R₁, R₂, R₃, and B and B'.

Although the present invention has been described with reference to the specific details of particular embodiments thereof, it is not intended that such details be regarded as limitations upon the scope of the invention except as and to the extent that they are included in the accompanying claims.

## Claims

1. A naphthopyran compound represented by the following graphic formula: 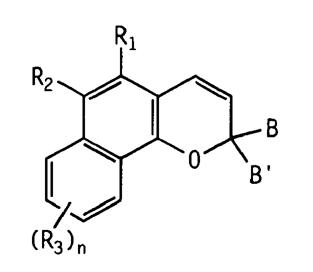 wherein,
(a) R₁ is the group, -C(O)W, W being -OR₄ or -N(R₅)R₆, wherein R₄ is hydrogen, allyl, C₁-C₆ alkyl, phenyl, mono(C₁-C₆)alkyl substituted phenyl, mono(C₁-C₆)alkoxy substituted phenyl, phenyl(C₁-C₃)alkyl, mono(C₁-C₆) alkyl substituted phenyl (C₁-C₃)alkyl, mono(C₁-C₆)alkoxy substituted phenyl (C₁-C₃)alkyl, C₁-C₆ alkoxy(C₂-C₄)alkyl, or C₁-C₆ haloalkyl; R₅ and R₆ are each selected from the group consisting of hydrogen, C₁-C₆ alkyl, C₅-C₇ cycloalkyl, phenyl and mono- or di-substituted phenyl, said phenyl substituents being selected from C₁-C₆ alkyl and C₁-C₆ alkoxy, and said halo substituent being chloro or fluoro;
(b) R₂ and each R₃ are hydrogen, C₁-C₆ alkyl, C₃-C₇ cycloalkyl, substituted or unsubstituted phenyl, the group -OR₇, wherein R₇ is hydrogen, C₁-C₆ alkyl, phenyl(C₁-C₃)alkyl, mono(C₁-C₆) alkyl substituted phenyl (C₁-C₃)alkyl, mono(C₁-C₆)alkoxy substituted phenyl (C₁-C₃)alkyl, C₁-C₆ alkoxy(C₂-C₄)alkyl, C₃-C₇ cycloalkyl, mono(C₁-C₄)alkyl substituted C₃-C₇ cycloalkyl, C₁-C₆ haloalkyl, allyl, the group, -CH(R₈)X, wherein X is -CN, -CF₃, halogen, or -C(O)W and R₈ is hydrogen or C₁-C₆ alkyl; or R₇ is the group, -C(O)Y, wherein Y is hydrogen or C₁-C₆ alkyl, C₁-C₆ alkoxy, the substituted or unsubstituted aryl groups phenyl or naphthyl, phenoxy, C₁-C₆ mono- or di-alkyl substituted phenoxy, C₁-C₆ alkylamino, phenylamino, C₁-C₆ mono- or di-alkyl substituted phenylamino, or C₁-C₆ mono- or di-alkoxy substituted phenylamino, each of said phenyl and naphthyl substituents being selected from C₁-C₆ alkyl or C₁-C₆ alkoxy, said halogen or halo substituents being chloro or fluoro, and n is selected from the integers 0, 1, 2 or 3; and
(c) B is selected from the group consisting of unsubstituted, mono-, di- and tri-substituted aryl groups phenyl and naphthyl, said aryl substituents being selected from the group consisting of C₁-C₆ alkyl, C₁-C₆ alkoxy, halogen, amino, C₁-C₆ monoalkylamino, C₁-C₆ dialkylamino, morpholino, piperidino, indolinyl, 1-imidazolidyl, 2-imidazolin-1-yl, 2-pyrazolidyl, pyrazolinyl, 1-piperazinyl and pyrrolidyl, said halogen being chloro or fluoro;
(d) B' is selected from the group consisting of:
mono-substituted aryl groups phenyl and naphthyl, said aryl substituents being selected from the group consisting of amino, C₁-C₆ monoalkylamino, C₁-C₆ dialkylamino, morpholino, piperidino, indolinyl, 1-imidazolidyl, 2-imidazolin-1-yl, 2-pyrazolidyl, pyrazolinyl, 1-piperazinyl and pyrrolidyl.

2. The naphthopyran of Claim 1, wherein:
(a) R₁ is the group, -C(O)W, W being -OR₄ or -N(R₅)R₆, wherein R₄ is hydrogen, C₁-C₄ alkyl, phenyl, mono(C₁-C₄)alkyl substituted phenyl, mono(C₁-C₄)alkoxy substituted phenyl, phenyl(C₁-C₂)alkyl, mono(C₁-C₄) alkyl substituted phenyl (C₁-C₂)alkyl, mono(C₁-C₄)alkoxy substituted phenyl (C₁-C₂)alkyl, mono(C₁-C₄)alkoxy(C₂-C₃)alkyl, or C₁-C₄ haloalkyl, and wherein R₅ and R₆ are each selected from the group consisting of hydrogen, C₁-C₄ alkyl, C₅-C₇ cycloalkyl, phenyl and mono- or di-substituted phenyl, said phenyl substituents being selected from C₁-C₄ alkyl and C₁-C₄ alkoxy, said halo substituent being chloro or fluoro;
(b) R₂ and each R₃ are hydrogen, C₁-C₃ alkyl, C₃-C₅ cycloalkyl, substituted or unsubstituted phenyl, or -OR₇, wherein R₇ is hydrogen, C₁-C₃ alkyl, or the group, CH(R₈)X, wherein X is -CN, or -C(O)W and R₈ is hydrogen or methyl, or R₇ is the group, -C(O)Y, wherein Y is C₁-C₃ alkyl or C₁-C₃ alkoxy, said phenyl substituents being C₁-C₃ alkyl or C₁-C₃ alkoxy, and n is selected from the integers 0 and 1; and
(c) B is selected from the group consisting of unsubstituted, mono- and di-substituted phenyl, said phenyl substituents being selected from the group consisting of C₁-C₄ alkyl, C₁-C₄ alkoxy, halogen, amino, C₁-C₄ monoalkylamino, C₁-C₄ dialkylamino, morpholino, piperidino, indolinyl and pyrrolidyl, said halogen being fluoro or chloro;
(d) B' is selected from the group consisting of mono-substituted phenyl, said phenyl substituents being selected from the group consisting of amino, C₁-C₄ monoalkylamino, C₁-C₄ dialkylamino, morpholino, piperidino, indolinyl and pyrrolidyl.

3. The naphthopyran of Claim 2, wherein:
(a) R₁ is the group, -C(O)W, W being -OR₄ or -N(R₅)R₆, wherein R₄ is hydrogen, C₁-C₃ alkyl, phenyl, mono(C₁-C₃)alkyl substituted phenyl, mono(C₁-C₃)alkoxy substituted phenyl, mono(C₁-C₃)alkoxy (C₂-C₃)alkyl, or C₁-C₃ haloalkyl, and wherein R₅ and R₆ are each selected from the group consisting of hydrogen, C₁-C₃ alkyl, C₅-C₇ cycloalkyl, phenyl and mono-substituted phenyl, said phenyl substituents being selected from C₁-C₃ alkyl and C₁-C₃ alkoxy, said halo substituent being fluoro;
(b) R₂ and R₃ are each selected from the group consisting of hydrogen, C₁-C₃ alkyl, phenyl and -OR₇, wherein R₇ is hydrogen or C₁-C₃ alkyl, or R₇ is the group, -C(O)Y, wherein Y is C₁-C₃ alkyl or C₁-C₃ alkoxy, and n is selected from the integers 0 and 1; and
(c) B is selected from the group consisting of unsubstituted, mono- and di-substituted phenyl, said phenyl substituents being selected from the group consisting of C₁-C₃ alkyl, C₁-C₃ alkoxy, fluoro, amino, C₁-C₃ monoalkylamino, C₁-C₃ dialkylamino, morpholino and piperidino;
(d) B' is selected from the group consisting of mono-substituted phenyl, said phenyl substituents being selected from the group consisting of amino, C₁-C₃ monoalkylamino, C₁-C₃ dialkylamino, morpholino and piperidino.

4. The naphthopyran of Claim 3, wherein R₁ is the group, -C(O)W, W being -OR₄ or -N(R₅)R₆, wherein R₄ is hydrogen or C₁-C₃ alkyl, R₅ and R₆ are each hydrogen, C₁-C₃ alkyl or phenyl; R₂ and R₃ are each hydrogen or C₁-C₃ alkyl; and B is an unsubstituted, mono-substituted or di-substituted phenyl, said phenyl substituents being C₁-C₂ alkyl, C₁-C₂ alkoxy, fluoro, C₁-C₂ monoalkylamino, C₁-C₂ dialkylamino, morpholino or piperidino; and B' is a mono-substituted phenyl, said phenyl substituents being C₁-C₂ monoalkylamino, C₁-C₂ dialkylamino, morpholino, or piperidino.

5. A naphthopyran compound selected form the group consisting of:
(a) 2-(4-morpholinophenyl)-2-phenyl-5-methoxycarbonyl-6-hydroxy-2H-naphtho[1,2-b]pyran;
(b) 2-(4-morpholinophenyl)-2-phenyl-5-methoxycarbonyl-6-methoxy-2H-naphtho[1,2-b]pyran;
(c) 2-(4-morpholinophenyl)-2-phenyl-5-methoxycarbonyl-6-acetoxy-2H-naphtho[1,2-b]pyran;
(d) 2-(4-dimethylaminophenyl)-2-phenyl-5-methoxycarbonyl-6-acetoxy-2H-naphtho[1,2-b]pyran;
(e) 2-(4-dimethylaminophenyl)-2-phenyl-5-methoxycarbonyl-6-methyl-2H-naphtho[1,2-b]pyran; and
(f) 2-2-bis(4-dimethylaminophenyl)-5-methoxycarbonyl-6-acetoxy-2H-naphtho[1,2-b]pyran.

6. A photochromic article comprising a polymeric organic host material and a photochromic amount of a naphthopyran compound of any of claims 1-5.

7. The photochromic article of Claim 6 wherein the photochromic compound is present in an amount of from 0.05 to 1.0 milligram per square centimeter of organic host material surface to which the photochromic substance(s) is incorporated or applied.

8. A photochromic article comprising, in combination, a solid transparent polymeric organic host material, and a photochromic amount of each of (a) at least one naphthopyran compound of any of claims 1-5, and (b) at least one other organic photochromic compound having at least one activated absorption maxima within the range of between 400 and 700 nanometers.

9. The photochromic article of claim 8 wherein the organic photochromic compound (b) is selected from the group consisting of:
(a) organic photochromic substances having at least one absorption maximum in the visible range of between 400 and less than 500 nanometers;
(b) organic photochromic substances having an absorption maximum within the visible range of between 400 and 500 nanometers and an absorption maximum within the visible range of between 500 and 700 nanometers; and
(c) organic photochromic substances having an activated absorption maxima in the visible range of greater than 570 nanometers; and
(d) mixtures of said organic photochromic substances.

10. The photochromic article of claim 9 wherein the organic photochromic compound (b) is an organic photochromic substances having an activated absorption maxima in the visible range of greater than 570 nanometers

11. The photochromic article of claim 9 wherein the organic photochromic compound (b) is selected from the group consisting of
spiro(indoline)naphthoxazines, spiro(indoline)-pyridobenzoxazines,
spiro(benzindoline)-pyridobenzoxazines,
spiro(benzindoline)naphthoxazines, spiro(benzindoline)naphthopyrans,
spiro(indoline)benzoxazines, spiro(indoline)benzopyrans,
spiro(indoline)naphthopyrans, spiro(indoline)quinopyrans,
spiro(indoline)pyrans, 3H-naphtho[2,1-b]pyrans, 2H-phenanthro[4,3-b]pyrans; 3H-phenanthro[1,2-b]pyrans; benzopyran compounds and mixtures of such photochromic substances.

12. The photochromic article of any of claims 6-11 wherein the polymeric organic host material is selected from the group consisting of polyacrylates, polymethacrylates, poly(C₁-C₁₂ alkyl methacrylates), polyoxy(alkylene methacrylates), poly (alkoxylated phenol methacrylates), cellulose acetate, cellulose triacetate, cellulose acetate propionate, cellulose acetate butyrate, poly(vinyl acetate), poly(vinyl alcohol), poly(vinyl chloride), poly(vinylidene chloride), thermoplastic polycarbonates, polyesters, polyurethanes, poly(ethylene terephthalate), polystyrene, poly(alpha methylstyrene), copoly(styrene-methylmethacrylate), copoly(styreneacrylonitrile), polyvinylbutyral and polymers of members of the group consisting of polyol(allyl carbonate) monomers, polyfunctional acrylate monomers, polyfunctional methacrylate monomers, diethylene glycol dimethacrylate monomers, diisopropenyl benzene monomers, alkoxylated polyhydric alcohol acrylate monomers and diallylidene pentaerythritol monomers.

13. The photochromic article of Claim 12 wherein the polymeric organic host material is a solid transparent homopolymer or copolymer selected from the group consisting of poly(methyl methacrylate), poly(ethylene glycol bis methacrylate), poly(ethoxylated bisphenol A dimethacrylate), thermoplastic polycarbonate, poly(vinyl acetate), polyvinylbutyral, polyurethane and polymers of members of the group consisting of diethylene glycol bis(allyl carbonate) monomers, diethylene glycol dimethacrylate monomers, diisopropenyl benzene monomers, and ethoxylated trimethylol propane triacrylate monomers.

14. The photochromic article of any of claims 6-13 wherein the article is a lens.

## Patentansprüche

1. Naphthopyranverbindung, die durch die folgende graphische Formel wiedergegeben ist: 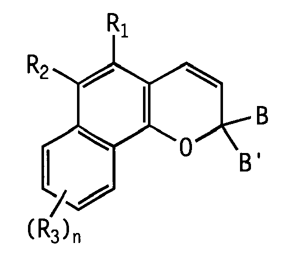 worin
(a) R₁ die Gruppe -C(O)W ist, wobei W gleich -OR₄ oder -N(R₅)R₆ ist, worin R₄ gleich Wasserstoff, Allyl, C₁-C₆-Alkyl, Phenyl, mono(C₁-C₆)-alkylsubstituiertes Phenyl, mono(C₁-C₆)alkoxysubstituiertes Phenyl, Phenyl(C₁-C₃)alkyl, mono(C₁-C₆)alkylsubstituiertes Phenyl(C₁-C₃)-alkyl, mono(C₁-C₆)alkoxysubstituiertes Phenyl(C₁-C₃)alkyl, C₁-C₆-Alkoxy(C₂-C₄)alkyl oder C₁-C₆-Halogenalkyl ist, R₅ und R₆ jeweils ausgewählt sind aus der Gruppe bestehend aus Wasserstoff, C₁-C₆-Alkyl, C₅-C₇-Cycloalkyl, Phenyl und mono- oder disubstituiertem Phenyl, wobei diese Substituenten des Phenyls ausgewählt sind aus C₁-C₆-Alkyl und C₁-C₆-Alkoxy und dieser Halogensubstituent Chlor oder Fluor ist,
(b) R₂ und jedes R₃ Wasserstoff, C₁-C₆-Alkyl, C₃-C₇-Cycloalkyl, substituiertes oder unsubstituiertes Phenyl, die Gruppe -OR₇ sind, worin R₇ gleich Wasserstoff, C₁-C₆-Alkyl, Phenyl(C₁-C₃)alkyl, mono(C₁-C₆)alkylsubstituiertes Phenyl(C₁-C₃)alkyl, mono(C₁-C₆)-alkoxysubstituiertes Phenyl(C₁-C₃)alkyl, C₁-C₆-Alkoxy(C₂-C₄)alkyl, C₃-C₇-Cycloalkyl, mono(C₁-C₄)alkylsubstituiertes C₃-C₇-Cycloalkyl, C₁-C₆-Halogenalkyl, Allyl, die Gruppe -CH(R₈)X ist, worin X gleich -CN, -CF₃, Halogen oder -C(O)W ist und R₈ gleich Wasserstoff oder C₁-C₆-Alkyl ist, oder R₇ die Gruppe -C(O)Y ist, worin Y gleich Wasserstoff oder C₁-C₆-Alkyl, C₁-C₆-Alkoxy, die substituierten oder unsubstituierten Arylgruppen Phenyl oder Naphthyl, Phenoxy, C₁-C₆-mono- oder dialkylsubstituiertes Phenoxy, C₁-C₆-Alkylamino, Phenylamino, C₁-C₆-mono- oder dialkylsubstituiertes Phenylamino oder C₁-C₆-mono- oder dialkoxysubstituiertes Phenylamino ist, wobei jeder dieser Substituenten des Phenyls und Naphthyls ausgewählt ist aus C₁-C₆-Alkyl oder C₁-C₆-Alkoxy und dieses Halogen oder diese Halogensubstituenten Chlor oder Fluor sind und n ausgewählt ist aus den ganzen Zahlen 0, 1, 2 oder 3, und
(c) B ausgewählt ist aus der Gruppe bestehend aus unsubstituierten, mono-, di- und trisubstituierten Arylgruppen Phenyl und Naphthyl, wobei diese Substituenten des Aryls ausgewählt sind aus der Gruppe bestehend aus C₁-C₆-Alkyl, C₁-C₆-Alkoxy, Halogen, Amino, C₁-C₆-Monoalkylamino, C₁-C₆-Dialkylamino, Morpholino, Piperidino, Indolinyl, 1-Imidazolidyl, 2-Imidazolin-1-yl, 2-Pyrazolidyl, Pyrazolinyl, 1-Piperazinyl und Pyrrolidyl, wobei dieses Halogen Chlor oder Fluor ist,
(d) B' ausgewählt ist aus der Gruppe bestehend aus monosubstituierten Arylgruppen Phenyl und Naphthyl, wobei diese Substituenten des Aryls ausgewählt sind aus der Gruppe bestehend aus Amino, C₁-C₆-Monoalkylamino, C₁-C₆-Dialkylamino, Morpholino, Piperidino, Indolinyl, 1-Imidazolidyl, 2-Imidazolin-1-yl, 2-Pyrazolidyl, Pyrazolinyl, 1-Piperazinyl und Pyrrolidyl.

2. Naphthopyran nach Anspruch 1, worin
(a) R₁ die Gruppe -C(O)W ist, wobei W gleich -OR₄ oder -N(R₅)R₆ ist, worin R₄ gleich Wasserstoff, C₁-C₄-Alkyl, Phenyl, mono(C₁-C₄)alkylsubstituiertes Phenyl, mono(C₁-C₄)alkoxysubstituiertes Phenyl, Phenyl(C₁-C₂)alkyl, mono(C₁-C₄)alkylsubstituiertes Phenyl(C₁-C₂)-alkyl, mono(C₁-C₄)alkoxysubstituiertes Phenyl(C₁-C₂)alkyl, Mono(C₁-C₄)alkoxy(C₂-C₃)alkyl oder C₁-C₄-Halogenalkyl ist und worin R₅ und R₆ jeweils ausgewählt sind aus der Gruppe bestehend aus Wasserstoff, C₁-C₄-Alkyl, C₅-C₇-Cycloalkyl, Phenyl und mono- oder disubstituiertem Phenyl, wobei diese Substituenten des Phenyls ausgewählt sind aus C₁-C₄-Alkyl und C₁-C₄-Alkoxy und dieser Halogensubstituent Chlor oder Fluor ist,
(b) R₂ und jedes R₃ gleich Wasserstoff, C₁-C₃-Alkyl, C₃-C₅-Cycloalkyl, substituiertes oder unsubstituiertes Phenyl oder -OR₇ sind, worin R₇ gleich Wasserstoff, C₁-C₃-Alkyl oder die Gruppe CH(R₈)X ist, worin X gleich -CN oder -C(O)W ist und R₈ gleich Wasserstoff oder Methyl ist oder R₇ die Gruppe -C(O)Y ist, worin Y gleich C₁-C₃-Alkyl oder C₁-C₃-Alkoxy ist, wobei diese Substituenten des Phenyls C₁-C₃-Alkyl oder C₁-C₃-Alkoxy sind und n ausgewählt ist aus den ganzen Zahlen 0 und 1, und
(c) B ausgewählt ist aus der Gruppe bestehend aus unsubstituiertem, mono- und disubstituiertem Phenyl, wobei diese Substituenten des Phenyls ausgewählt sind aus der Gruppe bestehend aus C₁-C₄-Alkyl, C₁-C₄-Alkoxy, Halogen, Amino, C₁-C₄-Monoalkylamino, C₁-C₄-Dialkylamino, Morpholino, Piperidino, Indolinyl und Pyrrolidyl, wobei dieses Halogen Fluor oder Chlor ist,
(d) B' ausgewählt ist aus der Gruppe bestehend aus monosubstituiertem Phenyl, wobei diese Substituenten des Phenyls ausgewählt sind aus der Gruppe bestehend aus Amino, C₁-C₄-Monoalkylamino, C₁-C₄-Dialkylamino, Morpholino, Piperidino, Indolinyl und Pyrrolidyl.

3. Naphthopyran nach Anspruch 2, worin
(a) R₁ die Gruppe -C(O)W ist, wobei W gleich -OR₄ oder -N(R₅)R₆ ist, worin R₄ gleich Wasserstoff, C₁-C₃-Alkyl, Phenyl, mono(C₁-C₃)alkylsubstituiertes Phenyl, mono(C₁-C₃)alkoxysubstituiertes Phenyl, Mono(C₁-C₃)alkoxy(C₂-C₃)alkyl oder C₁-C₃-Halogenalkyl ist und worin R₅ und R₆ jeweils ausgewählt sind aus der Gruppe bestehend aus Wasserstoff, C₁-C₃-Alkyl, C₅-C₇-Cycloalkyl, Phenyl und monosubstituiertem Phenyl, wobei diese Substituenten des Phenyls ausgewählt sind aus C₁-C₃-Alkyl und C₁-C₃-Alkoxy und dieser Halogensubstituent gleich Fluor ist,
(b) R₂ und R₃ jeweils ausgewählt sind aus der Gruppe bestehend aus Wasserstoff, C₁-C₃-Alkyl, Phenyl und -OR₇, worin R₇ gleich Wasserstoff oder C₁-C₃-Alkyl ist oder R₇ die Gruppe -C(O)Y ist, worin Y gleich C₁-C₃-Alkyl oder C₁-C₃-Alkoxy ist und n ausgewählt ist aus den ganzen Zahlen 0 und 1, und
(c) B ausgewählt ist aus der Gruppe bestehend aus unsubstituiertem, mono- und disubstituiertem Phenyl, wobei diese Substituenten des Phenyls ausgewählt sind aus der Gruppe bestehend aus C₁-C₃-Alkyl, C₁-C₃-Alkoxy, Fluor, Amino, C₁-C₃-Monoalkylamino, C₁-C₃-Dialkylamino, Morpholino und Piperidino,
(d) B' ausgewählt ist aus der Gruppe bestehend aus monosubstituiertem Phenyl, wobei diese Substituenten des Phenyls ausgewählt sind aus der Gruppe bestehend aus Amino, C₁-C₃-Monoalkylamino, C₁-C₃-Dialkylamino, Morpholino und Piperidino.

4. Naphthopyran nach Anspruch 3, worin R₁ die Gruppe -C(O)W ist, wobei W gleich -OR₄ oder -N(R₅)R₆ ist, worin R₄ gleich Wasserstoff oder C₁-C₃-Alkyl ist, R₅ und R₆ jeweils Wasserstoff, C₁-C₃-Alkyl oder Phenyl sind, R₂ und R₃ jeweils Wasserstoff oder C₁-C₃-Alkyl sind und B ein unsubstituiertes, monosubstituiertes oder disubstituiertes Phenyl ist, wobei diese Substituenten des Phenyls C₁-C₂-Alkyl, C₁-C₂-Alkoxy, Fluor, Amino, C₁-C₂-Monoalkylamino, C₁-C₂-Dialkylamino, Morpholino oder Piperidino sind und B' ein monosubstituiertes Phenyl ist, wobei diese Substituenten des Phenyls C₁-C₂-Monoalkylamino, C₁-C₂-Dialkylamino, Morpholino oder Piperidino sind.

5. Naphthopyranverbindung, ausgewählt aus der Gruppe bestehend aus:
(a) 2-(4-Morpholinophenyl)-2-phenyl-5-methoxycarbonyl-6-hydroxy-2H-naphtho[1,2-b]pyran,
(b) 2-(4-Morpholinophenyl)-2-phenyl-5-methoxycarbonyl-6-methoxy-2H-naphtho[1,2-b]pyran,
(c) 2-(4-Morpholinophenyl)-2-phenyl-5-methoxycarbonyl-6-acetoxy-2H-naphtho[1,2-b]pyran,
(d) 2-(4-Dimethylaminophenyl)-2-phenyl-5-methoxycarbonyl-6-acetoxy-2H-naphtho[1,2-b]pyran,
(e) 2-(4-Dimethylaminophenyl)-2-phenyl-5-methoxycarbonyl-6-methyl-2H-naphtho[1,2-b]pyran und
(f) 2-2-Bis(4-dimethylaminophenyl)-5-methoxycarbonyl-6-acetoxy-2H-naphtho[1,2-b]pyran.

6. Photochromer Gegenstand, enthaltend ein polymeres organisches Wirtsmaterial und eine photochrome Menge einer Naphthopyranverbindung nach einem der Ansprüche 1-5.

7. Photochromer Gegenstand nach Anspruch 6, wobei die photochrome Verbindung in einer Menge von 0,05 bis 1,0 mg/cm² organischer Wirtsmaterialoberfläche vorhanden ist, auf die die photochrome Substanz(en) eingebracht oder aufgebracht ist(sind).

8. Photochromer Gegenstand, enthaltend in Kombination ein festes transparentes polymeres organisches Wirtsmaterial und eine photochrome Menge von jeweils (a) wenigstens einer Naphthopyranverbindung nach einem der Ansprüche 1-5 und (b) wenigstens einer anderen organischen photochromen Verbindung mit wenigstens einem aktivierten Absorptionsmaximum im Bereich zwischen 400 und 700 nm.

9. Photochromer Gegenstand nach Anspruch 8, wobei die organische photochrome Verbindung (b) ausgewählt ist aus der Gruppe bestehend aus:
(a) organischen photochromen Substanzen mit wenigstens einem Absorptionsmaximum im sichtbaren Bereich zwischen 400 und weniger als 500 nm,
(b) organischen photochromen Substanzen mit einem Absorptionsmaximum im sichtbaren Bereich zwischen 400 und 500 nm und einem Absorptionsmaximum im sichtbaren Bereich zwischen 500 und 700 nm und
(c) organischen photochromen Substanzen mit einem aktivierten Absorptionsmaximum im sichtbaren Bereich von mehr als 570 nm und
(d) Mischungen dieser organischen photochromen Substanzen.

10. Photochromer Gegenstand nach Anspruch 9, wobei die organische photochrome Verbindung (b) eine organische photochrome Substanz mit einem aktivierten Absorptionsmaximum im sichtbaren Bereich von mehr als 570 nm ist.

11. Photochromer Gegenstand nach Anspruch 9, wobei die organische photochrome Verbindung (b) ausgewählt ist aus der Gruppe bestehend aus Spiro(indolin)naphthoxazinen, Spiro(indolin)pyridobenzoxazinen, Spiro(benzindolin)pyridobenzoxazinen, Spiro(benzindolin)naphthoxazinen, Spiro(benzindolin)naphthopyranen, Spiro(indolin)benzoxazinen, Spiro(indolin)benzopyranen, Spiro(indolin)naphthopyranen, Spiro(indolin)chinopyranen, Spiro(indolin)pyranen, 3H-Naphtho[2,1-b]-pyranen, 2H-Phenanthro[4,3-b]pyranen, 3H-Phenanthro[1,2-b]pyranen, Benzopyranverbindungen und Mischungen solcher photochromen Substanzen.

12. Photochromer Gegenstand nach einem der Ansprüche 6-11, wobei das polymere organische Wirtsmaterial ausgewählt ist aus der Gruppe bestehend aus Polyacrylaten, Polymethacrylaten, Poly(C₁-C₁₂-alkylmethacrylaten), Polyoxy(alkylenmethacrylaten), poly(alkoxylierten Phenolmethacrylaten), Celluloseacetat, Cellulosetriacetat, Celluloseacetatpropionat, Celluloseacetatbutyrat, Poly(vinylacetat), Poly(vinylalkohol), Poly(vinylchlorid), Poly(vinylidenchlorid), thermoplastischen Polycarbonaten, Polyestern, Polyurethanen, Poly(ethylenterephthalat), Polystyrol, Poly(α-methylstyrol), Copoly(styrol-methylmethacrylat), Copoly(styrol-acrylnitril), Polyvinylbutyral und Polymeren der Mitglieder der Gruppe bestehend aus Polyol(allylcarbonat)monomeren, polyfunktionellen Acrylatmonomeren, polyfunktionellen Methacrylatmonomeren, Diethylenglykoldimethacrylatmonomeren, Diisopropenylbenzolmonomeren, alkoxylierten Polyhydroxyalkoholacrylatmonomeren und Diallylidenpentaerythritmonomeren.

13. Photochromer Gegenstand nach Anspruch 12, wobei das polymere organische Wirtsmaterial ein festes transparentes Homopolymer oder Copolymer ist, ausgewählt aus der Gruppe bestehend aus Poly(methylmethacrylat), Poly(ethylenglykolbismethacrylat), poly(ethoxyliertem Bisphenol-A-dimethacrylat), thermoplastischem Polycarbonat, Poly(vinylacetat), Polyvinylbutyral, Polyurethan und Polymeren der Mitglieder der Gruppe bestehend aus Diethylenglykolbis-(allylcarbonat)monomeren, Diethylenglykoldimethacrylatmonomeren, Diisopropenylbenzolmonomeren und ethoxylierten Trimethylolpropantriacrylatmonomeren.

14. Photochromer Gegenstand nach einem der Ansprüche 6-13, wobei der Gegenstand eine Linse ist.

## Revendications

1. Composé de naphtopyranne représenté par la formule graphique suivante : 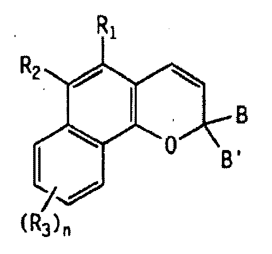 dans laquelle
(a) R₁ est le groupe -C(O)W, W étant un groupe -OR₄ ou -N(R₅)R₆, dans lequel R₄ est de l'hydrogène, un groupe allyle, alkyle en C₁-C₆, phényle, phényle substitué par monoalkyle en C₁-C₆, phényle substitué par monoalcoxy en C₁-C₆, phénylalkyle en C₁-C₃, phénylalkyle en C₁-C₃ substitué par monoalkyle en C₁-C₆, phénylalkyle en C₁-C₃ substitué par monoalcoxy en C₁-C₆, alcoxy(C₁-C₆)alkyle en C₂-C₄ ou haloalkyle en C₁-C₆; R₅ et R₆ sont chacun choisis dans le groupe comprenant l'hydrogène, les groupes alkyle en C₁-C₆, cycloalkyle en C₅-C₇, phényle et phényle mono- ou disubstitués, lesdits substituants de phényle étant choisis parmi les groupes alkyle en C₁-C₆ et alcoxy en C₁-C₆, et ledit substituant halo étant du chloro ou du fluoro;
(b) R₂ et chaque R₃ sont de l'hydrogène, un groupe alkyle en C₁-C₆, cycloalkyle en C₃-C₇, phényle substitué ou non substitué, le group -OR₇, dans lequel R₇ est de l'hydrogène, un groupe alkyle en C₁-C₆, phénylalkyle en C₁-C₃, phénylalkyle en C₁-C₃ substitué par monoalkyle en C₁-C₆, phénylalkyle en C₁-C₃ substitué par monoalcoxy en C₁-C₆, alcoxy(C₁-C₆)alkyle en C₂-C₄, cycloalkyle en C₃-C₇, cycloalkyle en C₃-C₇ substitué par monoalkyle en C₁-C₄, haloalkyle en C₁-C₆, allyle, le groupe -CH(R₈)X, dans lequel X est -CN, -CF₃, un halogène ou un groupe -C(O)W et R₈ est de l'hydrogène ou un groupe alkyle en C₁-C₆; ou bien R₇ est le groupe -C(O)Y, dans lequel Y est de l'hydrogène ou un groupe alkyle en C₁-C₆, alcoxy en C₁-C₆, les groupes aryle substitués ou non substitués phényle ou naphtyle, les groupes phénoxy, phénoxy substitués par mono- ou dialkyle en C₁-C₆, alkylamino en C₁-C₆, phénylamino, phénylamino substitués par mono- ou dialkyle en C₁-C₆ ou phénylamino substitués par mono- ou dialcoxy en C₁-C₆, chacun desdits substituants de phényle et naphtyle étant choisi parmi les groupes alkyle en C₁-C₆ ou alcoxy en C₁-C₆, lesdits halogène ou substituants halo étant du chloro ou du fluoro, et n est choisi parmi les nombres entiers 0, 1, 2 et 3; et
(c) B est choisi dans le groupe comprenant les groupes aryle non substitués, mono-, di- et trisubstitués phényle et naphtyle, lesdits substituants d'aryle étant choisis dans le groupe comprenant les groupes alkyle en C₁-C₆, alcoxy en C₁-C₆, halogène, amino, monoalkyl(C₁-C₆)-amino, dialkyl(C₁-C₆)amino, morpholino, pipéridino, indolinyle, 1-imidazolidyle, 2-imidazolin-1-yle, 2-pyrazolidyle, pyrazolinyle, 1-pipérazinyle et pyrrolidyle, ledit halogène étant du chloro ou du fluoro;
(d) B' est choisi dans le groupe comprenant les groupes aryle monosubstitués phényle et naphtyle, lesdits substituants d'aryle étant choisis dans le groupe comprenant les groupes amino, monoalkyl(C₁-C₆)amino, dialkyl(C₁-C₆)amino, morpholino, pipéridino, indolinyle, 1-imidazolidyle, 2-imidazolin-1-yle, 2-pyrazolidyle, pyrazolinyle, 1-pipérazinyle et pyrrolidyle.

2. Naphtopyranne suivant la revendication 1, dans lequel :
(a) R₁ est le groupe -C(O)W, W étant un groupe -OR₄ ou -N(R₅)R₆, dans lequel R₄ est de l'hydrogène, un groupe alkyle en C₁-C₄, phényle, phényle substitué par monoalkyle en C₁-C₄, phényle substitué par monoalcoxy en C₁-C₄, phénylalkyle en C₁-C₂, phénylalkyle en C₁-C₂ substitué par monoalkyle en C₁-C₄, phénylalkyle en C₁-C₂ substitué par monoalcoxy en C₁-C₄, monoalcoxy(C₁-C₄)alkyle en C₂-C₃ ou haloalkyle en C₁-C₄, et dans lequel R₅ et R₆ sont chacun choisis dans le groupe comprenant l'hydrogène, les groupes alkyle en C₁-C₄, cycloalkyle en C₅-C₇, phényle et phényle mono- ou disubstitués, lesdits substituants de phényle étant choisis parmi les groupes alkyle en C₁-C₄ et alcoxy en C₁-C₄, ledit substituant halo étant du chloro ou du fluoro;
(b) R₂ et chaque R₃ sont de l'hydrogène, un groupe alkyle en C₁-C₃, cycloalkyle en C₃-C₅, phényle substitué ou non substitué, ou -OR₇, dans lequel R₇ est de l'hydrogène, un groupe alkyle en C₁-C₃ ou le groupe CH(R₈)X, dans lequel X est un groupe -CN ou -C(O)W et R₈ est de l'hydrogène ou du méthyle, ou bien R₇ est le groupe -C(O)Y, dans lequel Y est un groupe alkyle en C₁-C₃ ou alcoxy en C₁-C₃, lesdits substituants de phényle étant un groupe alkyle en C₁-C₃ ou alcoxy en C₁-C₃, et n est choisi parmi les nombres entiers 0 et 1; et
(c) B est choisi dans le groupe comprenant les groupes phényle non substitué, mono- et disubstitués, lesdits substituants de phényle étant choisis dans le groupe comprenant les groupes alkyle en C₁-C₄, alcoxy en C₁-C₄, halogène, amino, monoalkyl(C₁-C₄)amino, dialkyl(C₁-C₄)amino, morpholino, pipéridino, indolinyle et pyrrolidyle, ledit halogène étant du fluoro ou du chloro;
(d) B' est choisi dans le groupe comprenant les groupes phényle monosubstitués, lesdits substituants de phényle étant choisis dans le groupe comprenant les groupes amino, monoalkyl(C₁-C₄)amino, dialkyl-(C₁-C₄)amino, morpholino, pipéridino, indolinyle et pyrrolidyle.

3. Naphtopyranne suivant la revendication 2, dans lequel :
(a) R₁ est le groupe -C(O)W, W étant un groupe -OR₄ ou -N(R₅)R₆, dans lequel R₄ est de l'hydrogène, un groupe alkyle en C₁-C₃, phényle, phényle substitué par monoalkyle en C₁-C₃, phényle substitué par monoalcoxy en C₁-C₃, monoalcoxy(C₁-C₃)alkyle en C₂-C₃ ou haloalkyle en C₁-C₃, et dans lequel R₅ et R₆ sont chacun choisis dans le groupe comprenant l'hydrogène, les groupes alkyle en C₁-C₃, cycloalkyle en C₅-C₇, phényle et phényle monosubstitués, lesdits substituants de phényle étant choisis parmi les groupes alkyle en C₁-C₃ et alcoxy en C₁-C₃, ledit substituant halo étant du fluoro;
(b) R₂ et R₃ sont chacun choisis dans le groupe comprenant l'hydrogène, les groupes alkyle en C₁-C₃, phényle et -OR₇, dans lequel R₇ est de l'hydrogène ou un groupe alkyle en C₁-C₃, ou bien R₇ est le groupe -C(O)Y, dans lequel Y est un groupe alkyle en C₁-C₃ ou alcoxy en C₁-C₃, et n est choisi parmi les nombres entiers 0 et 1 ; et
(c) B est choisi dans le groupe comprenant les groupes phényle non substitué, mono- et disubstitués, lesdits substituants de phényle étant choisis dans le groupe comprenant les groupes alkyle en C₁-C₃, alcoxy en C₁-C₃, fluoro, amino, monoalkyl(C₁-C₃)amino, dialkyl(C₁-C₃)amino, morpholino et pipéridino;
(d) B' est choisi dans le groupe comprenant les groupes phényle monosubstitués, lesdits substituants de phényle étant choisis dans le groupe comprenant les groupes amino, monoalkyl(C₁-C₃)amino, dialkyl-(C₁-C₃)amino, morpholino et pipéridino.

4. Naphtopyranne suivant la revendication 3, dans lequel R₁ est le groupe -C(O)W, W étant un groupe -OR₄ ou -N(R₅)R₆, dans lequel R₄ est de l'hydrogène ou un groupe alkyle en C₁-C₃, R₅ and R₆ sont chacun de l'hydrogène, un groupe alkyle en C₁-C₃ ou phényle; R₂ et R₃ sont chacun de l'hydrogène ou un groupe alkyle en C₁-C₃; et B est un groupe phényle non substitué, monosubstitué ou disubstitué, lesdits substituants de phényle étant un groupe alkyle en C₁-C₂, alcoxy en C₁-C₂, fluoro, monoalkyl(C₁-C₂)amino, dialkyl(C₁-C₂)amino, morpholino ou pipéridino; et B' est un groupe phényle monosubstitué, lesdits substituants de phényle étant un groupe monoalkyl(C₁-C₂)amino, dialkyl(C₁-C₂)amino, morpholino ou pipéridino.

5. Composé de naphtopyranne choisi dans le groupe comprenant :
(a) le 2-(4-morpholinophényl)-2-phényl-5-méthoxycarbonyl-6-hydroxy-2H-naphto[1,2-b]pyranne;
(b) le 2-(4-morpholinophényl)-2-phényl-5-méthoxycarbonyl-6-méthoxy-2H-naphto[1,2-b]pyranne;
(c) le 2-(4-morpholinophényl)-2-phényl-5-méthoxycarbonyl-6-acétoxy-2H-naphto[1,2-b]pyranne;
(d) le 2-(4-diméthylaminophényl)-2-phényl-5-méthoxycarbonyl-6-acétoxy-2H-naphto[1,2-b]pyranne;
(e) le 2-(4-diméthylaminophényl)-2-phényl-5-méthoxycarbonyl-6-méthyl-2H-naphto[1,2-b]pyranne, et
(f) le 2,2-bis(4-diméthylaminophényl)-5-méthoxycarbonyl-6-acétoxy-2H-naphto[1, 2-b]pyranne.

6. Article photochromique comprenant une matière hôte organique polymérique et une quantité photochromique d'un composé de naphtopyranne suivant l'une quelconque des revendications 1 à 5.

7. Article photochromique suivant la revendication 6, dans lequel le composé photochromique est présent en une quantité de 0,05 à 1,0 mg par cm² de surface de matière hôte organique à laquelle la ou les substances photochromiques sont incorporées ou appliquées.

8. Article photochromique comprenant, en combinaison, une matière hôte organique polymérique transparente solide, et une quantité photochromique de chacun (a) d'au moins un composé de naphtopyranne suivant l'une quelconque des revendications 1 à 5, et (b) d'au moins un autre composé photochromique organique ayant au moins un maximum d'absorption activé dans la plage entre 400 et 700 nanomètres.

9. Article photochromique suivant la revendication 8, dans lequel le composé photochromique organique (b) est choisi dans le groupe comprenant :
(a) les substances photochromiques organiques ayant au moins un maximum d'absorption dans la région visible entre 400 et moins de 500 nanomètres;
(b) les substances photochromiques organiques ayant un maximum d'absorption dans la région visible entre 400 et 500 nanomètres et un maximum d'absorption dans la région visible entre 500 et 700 nanomètres; et
(c) les substances photochromiques organiques ayant un maximum d'absorption activé dans la région visible de plus de 570 nanomètres; et
(d) les mélanges desdites substances photochromiques organiques.

10. Article photochromique suivant la revendication 9, dans lequel le composé photochromique organique (b) est une substance photochromique organique ayant un maximum d'absorption activé dans la région visible de plus de 570 nanomètres.

11. Article photochromique suivant la revendication 9, dans lequel le composé photochromique organique (b) est choisi dans le groupe comprenant les spiro(indoline)naphtoxazines, les spiro(indoline)pyridobenzoxazines, les spiro(benzindoline)pyridobenzoxazines, les spiro(benzindoline)naphtoxazines, les spiro(benzindoline)naphtopyrannes, les spiro-(indoline)benzoxazines, les spiro(indoline)benzopyrannes, les spiro-(indoline)naphtopyrannes, les spiro(indoline)quinopyrannes, les spiro-(indoline)pyrannes, les 3H-naphto[2,1-b]pyrannes, les 2H-phénanthro-[4,3-b]pyrannes, les 3H-phénanthro[1,2-b]pyrannes, les composés de benzopyranne et les mélanges de ces substances photochromiques.

12. Article photochromique suivant l'une quelconque des revendications 6 à 11, dans lequel la matière hôte organique polymérique est choisie dans le groupe comprenant les polyacrylates, les polyméthacrylates, les poly(méthacrylates d'alkyle en C₁-C₁₂), les polyoxy-(méthacrylates d'alkylène), les poly(méthacrylates phénoliques alcoxylés), l'acétate de cellulose, le triacétate de cellulose, l'acétatopropionate de cellulose, l'acétobutyrate de cellulose, le poly(acétate de vinyle), le poly(alcool vinylique), le poly(chlorure de vinyle), le poly(chlorure de vinylidène), les polycarbonates thermoplastiques, les polyesters, les polyuréthannes, le poly(éthylène téréphtalate), le polystyrène, le poly(alphaméthylstyrène), le copoly(styrène-méthacrylate de méthyle), le copoly-(styrène-acrylonitrile), le polyvinylbutyral et les polymères des membres du groupe comprenant les monomères de polyol(allyl carbonate), les monomères d'acrylate polyfonctionnels, les monomères de méthacrylate polyfonctionnels, les monomères de diéthylène glycol diméthacrylate, les monomères de diisopropényl benzène, les monomères d'acrylate d'alcool polyvalent alcoxylé et les monomères de diallylidène pentaérythritol.

13. Article photochromique suivant la revendication 12, dans lequel la matière hôte organique polymérique est un homopolymère ou copolymère transparent solide choisi dans le groupe comprenant le poly(méthacrylate de méthyle), le poly(bisméthacrylate d'éthylène glycol), le poly(diméthacrylate de bisphénol A éthoxylé), les polycarbonates thermoplastiques, le poly(acétate de vinyle), le polyvinylbutyral, les polyuréthannes et les polymères des membres du groupe comprenant les monomères de diéthylène glycol bis(allyl carbonate), les monomères de diéthylène glycol diméthacrylate, les monomères de diisopropényl benzène et les monomères de triméthylol propane triacrylate éthoxylé.

14. Article photochromique suivant l'une quelconque des revendications 6 à 13, dans lequel l'article est une lentille.
